# EUROPEAN PATENT SPECIFICATION

(11) **EP 2 055 341 B1**
(45) Date of publication and mention of the grant of the patent: **28.03.2012**
(21) Application number: 07425680.1
(22) Date of filing: 29.10.2007
(51) Int. Cl.: A61M 16/20, F16L 37/248

(54) **Connection device for connecting an apparatus for medical use to an end unit of a distribution plant**
Anschlussvorrichtung zum Verbinden eines Geräts für medizinische Zwecke mit einem Endanschluss einer Verteilungsanlage
Dispositif de raccordement pour raccorder un appareil pour utilisation médicale à un raccord d'extrémité d'une installation de distribution

(43) Date of publication of application: 06.05.2009
(73) Proprietor: Flow Meter S.p.a., 24040 Levate (Bergamo) (IT)
(72) Inventor: Paratico, Roberto, 24040 Levate (Bergamo) (IT)
(74) Representative: Botti, Mario

(56) References cited:
- WO-A-99/36722
- FR-A- 2 895 057
- US-A- 1 615 595
- US-A1- 2006 087 120

## Description

### Field of application

The present invention, in its most general aspect, refers to the field of the distribution plants of compressed gases for medical use or of a vacuum distribution plant, and in particular to an end unit of the distribution plant, also called intake unit, i.e. that unit mounted on a fixed or movable wall of a distribution plant on which a user operates repeated connections and detachments for drawing the compressed gases or vacuum and supplying a specific apparatus for medical use, such as for example an anaesthesia apparatus, assisted ventilation apparatus, breathing apparatus and the like.

More in particular, the present invention regards a connection device for connecting an apparatus for medical use to an end unit of a distribution plant of compressed gases or of a vacuum distribution plant in which the connection device is adapted to be fixed to the end unit by means of a bayonet clutch having a fixed part and a movable part, of which the end unit constitutes the fixed part and in which the connection device is of the type comprising a sleeve having a tubular body extended along a specific axis between a first end adapted to be associated with the end unit and a second end adapted to be associated with the apparatus for medical use for the passage of the compressed gas or the vacuum, and also having a fastening element constituting the movable part of the bayonet clutch, and a handle element associated with the fastening element.

### Connection device of this type is known from WO-A-99/36722,

For the sake of illustration simplicity, the following description is made with reference to an end supply unit. Of course, all that described and claimed below for an end supply unit is also valid for an end suction unit of a vacuum distribution plant, where the structural analogies with the end supply unit are evident.

It is moreover intended to specify that with the term apparatus for medical use, it is intended for example an apparatus which uses a specific gas for therapeutic purposes, a surgical instrument or the like, or a technical hospital apparatus, i.e. an apparatus which is used not for therapeutic purposes, but only for checking medical apparatuses, or for actuating surgical instruments, and the like.

### Prior Art

With reference to the abovementioned sector, the need is known for arranging a plurality of end units alongside each other on the wall of a room or of-a movable-trolley, or of a flexible unit wherein each end unit is destined for supplying a specific gas for medical use. This permits an operator to substantially connect, from the same station, a plurality of apparatuses for medical use using different gases, as needed.

For such purposes, a plurality of apparatuses for medical use are provided with related connection devices, each of which, as mentioned above, comprises a fastening element which constitutes the movable part of a bayonet clutch. The fastening element is subsequently fixed to the related end unit, which constitutes the fixed part of the bayonet clutch.

In one such joint, as is known, the movable part translates and rotates with respect to the fixed part, for the connection of the parts, and subsequently rotates and translates, for the release.

The rotation manoeuvre of the movable part, which occurs around the axis of the sleeve, is facilitated by the handle element, or grip, which in the known device is substantially shaped as a short tube.

While advantageous in many respects, and substantially satisfying the object, the known device nevertheless involves recognised drawbacks which have yet to be overcome.

In particular, the main drawback depends on the fact that at the time of fixing or disconnection, a rotation of the fastening element around the axis of the sleeve also causes an angular displacement of the apparatus for medical use. However, the angular displacement of the apparatus for medical use at the time of fixing or disconnection of the bayonet clutch can be obstructed, or even blocked, by the presence of the other, adjacent apparatuses set side-by-side each other.

For this reason, after the fixing of the bayonet clutch, it is necessary to newly position each apparatus in the correct position. For such purpose, it is necessary to provide a locking of removable type between the fastening element on one side, and the handle element and sleeve on the other,

In this manner, by momentarily freeing the restraint between the handle element and the fastening element, and by angularly moving the handle element, the apparatus can be angularly moved into the correct operative position, without interfering with the fixing of the bayonet clutch.

Therefore, as a further drawback, in order to reposition the apparatus into correct position, the known connection device requires carrying out the aforesaid additional release operation between the handle element and the fastening element. In order to carry out such operations, both hands of the operator are moreover required.

The technical problem underlying the present invention is that of devising a connection device having structure and functional characteristics such to permit overcoming the abovementioned drawbacks, and in particular which allow connecting an apparatus for medical use to the end unit in a simple manner, also in the presence of a plurality of end units arranged alongside each other, and possibly by using only one hand.

### Summary of the invention

The aforesaid problem is solved by a connection device according to claim 1.

The aforesaid technical problem is moreover solved by an apparatus for medical use according to claim 14 and by a compressed gas supply device according to claim 15.

The main advantage of the connection device according to the present invention lies in the fact that the fastening element and the handle element can be angularly moved around the sleeve axis, independent of the apparatus for medical use. In this manner, at the time of fixing of the fastening element to the end unit, the apparatus for medical use is not constrained in rotation.

In order to favour the manoeuvring of the fastening element, the handle element comprises an annular shell, which surrounds the fastening element.

Preferably, the shell has a substantially C-shaped section and is formed by a mantle, a first end section turned towards the first end of the sleeve, and a second end section having greater extension than the first end section, and turned towards the second end of the sleeve. The mantle, the first end section and the second end section define an inner annular recess in which a portion of the fastening element is housed.

Still more preferably, the handle element also acts as an axial stop of the fastening element on the sleeve. In particular, the fastening element is axially constrained between the first end of the sleeve bearing a circular flange, and the second end section of the shell, which is housed in a circular groove made on the outer surface of the sleeve.

The restraint between the shell and the fastening element is obtained by means of teeth and respective counter-teeth. The counter-teeth of the fastening element are arranged on the entire outer circumference of the latter, forming a toothed rim. In particular, in a preferred solution, the entire counter-teeth rim of the fastening element is housed and retained between the first end section and the second end section of the shell, in order to ensure a firm restraint between the two elements.

In order to favour the mounting of the shell on the fastening element, the shell is formed by two opposite half-shells having semicircular form.

Preferably, each half-shell has the same colour, or, in some cases, each has a different colour from the other, and bears a symbol or writing indicating the name of the compressed gas.

Further characteristics and advantages of the connection device according to the invention will be clearer from the following description of an embodiment thereof, given as indicative and non-limiting with reference to the attached drawings.

### Brief description of the drawings

In such drawings:
Fig. 1 is an axonometric view of a connection device according to the invention associated with an apparatus for medical use in a first connection step to an end unit;
Fig. 2 is an axonometric view of a connection device according to the invention associated with an apparatus for medical use in a second connection step to an end unit;

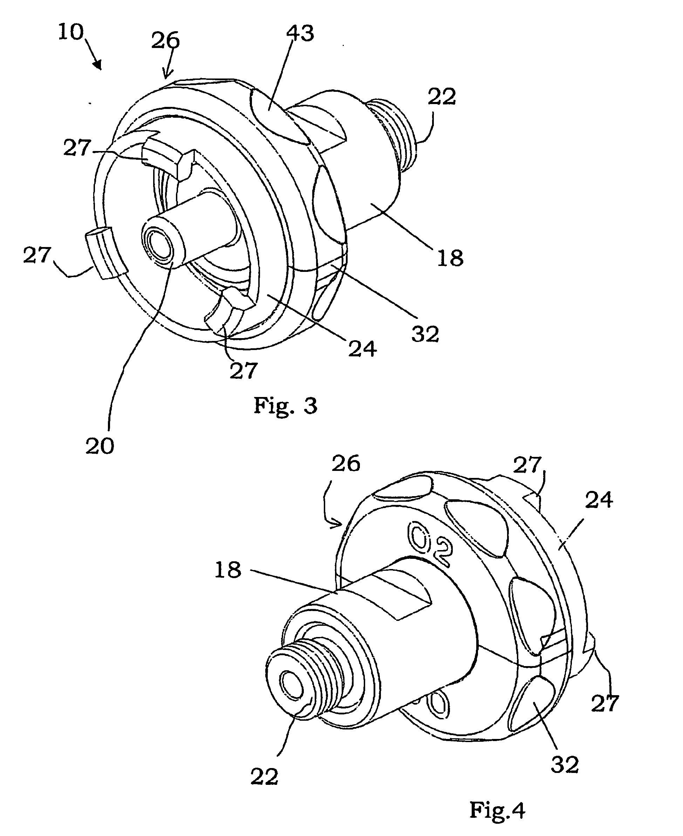

Fig. 3 is a front axonometric view of the connection device according to the invention;
Fig. 4 is a rear axonometric view of the connection device according to the invention;

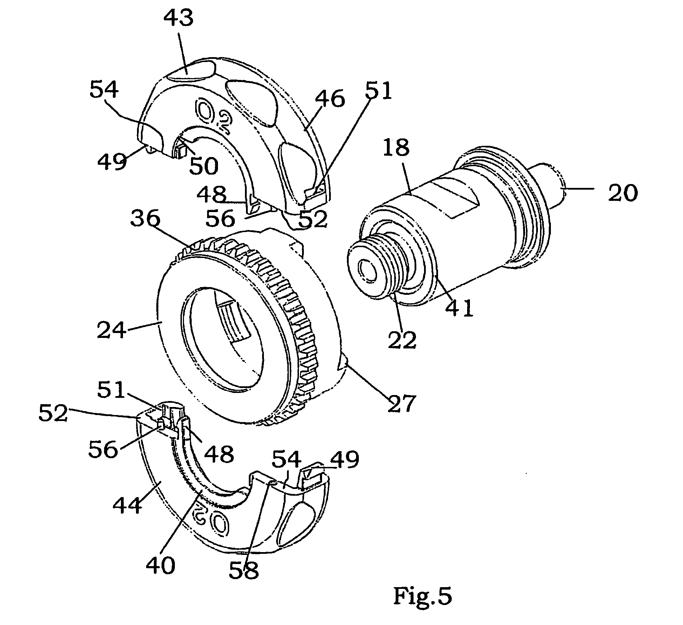

Fig. 5 is a view of the connection device according to the invention with separated parts.
Fig. 6 is a plan view of the connection device according to the invention;
Fig. 7 is a section taken along the line VI-VI of the connection device of figure 6.

### Detailed description

With reference to such figures, a connection device according to the invention is schematically indicated in its entirety with 10, the connection device 10 being associated with an apparatus for medical use 12, in the present case a flow meter for medical oxygen. The connection device 10 connects such apparatus for medical use 12 to an end supply unit 14 of compressed oxygen.

In particular, the device is adapted to be fixed to the end unit 14 by means of a bayonet clutch having a fixed part and a movable part, on which the end unit 14 constitutes the fixed part.

Still more in particular, the connection device 10 comprises a sleeve 18 having a tubular body extended along an X axis between a first end 20 adapted to be associated with the end unit 14 and a second end 22 adapted to be associated with the apparatus for medical use 12. In the illustrated solution, the first end 20 has substantially cylindrical form and is inserted in a corresponding hole 21 present in the end unit 14, while the second end 22 is provided with a thread so to be connected by means of screwing to the apparatus for medical use 12.

The sleeve 18 also has a fastening element 24, which is associated at the first end 20 of the sleeve 18, and constitutes the movable part of the bayonet clutch.

In particular, the fastening element 24 comprises a hollow body having substantially cylindrical form and supporting, at its free end turned towards the end unit 14, three front teeth 27 in order to permit the coupling to the end unit 14. The latter is constituted, in a known manner, by a cylindrical block having the aforesaid central hole 21 and provided on its outer surface with three grooves 31.

Each groove 31 has a substantially L-shaped, angular form, and is run through by the corresponding tooth 27 of the fastening element 24, by means of axial translation and angular displacement. Still more in particular, in the illustrated solution, each groove 31 has a further brief angular section formed at the free end of the L. The tooth 27 is positioned in such section after the angular movement, under the action of a spring, not visible in the drawings, such spring being inserted in the end unit 14 at the hole 21. Each tooth 27 stably remains in such position, preventing a release of the parts other than that expressly actuated by an authorised person, by overcoming the force of the spring.

The connection device 10 also comprises a handle element 26, which is associated with the fastening element 24, and acts as a grip for manoeuvring the latter in the device mounting or disassembly step.

According to one aspect of the present invention, the fastening element 24 and the handle element 26 form a separately manoeuvrable single body, rotatably and idly mounted on the sleeve 18. In substance, the fastening element 24 and the handle element 26 are rigidly bound to each other to form a separately manoeuvrable piece, freely rotatable with respect to the sleeve 18 around the X axis of the latter.

More in particular, the handle element 26 comprises an annular shell 32 made of plastic material, which surrounds the fastening element 24.

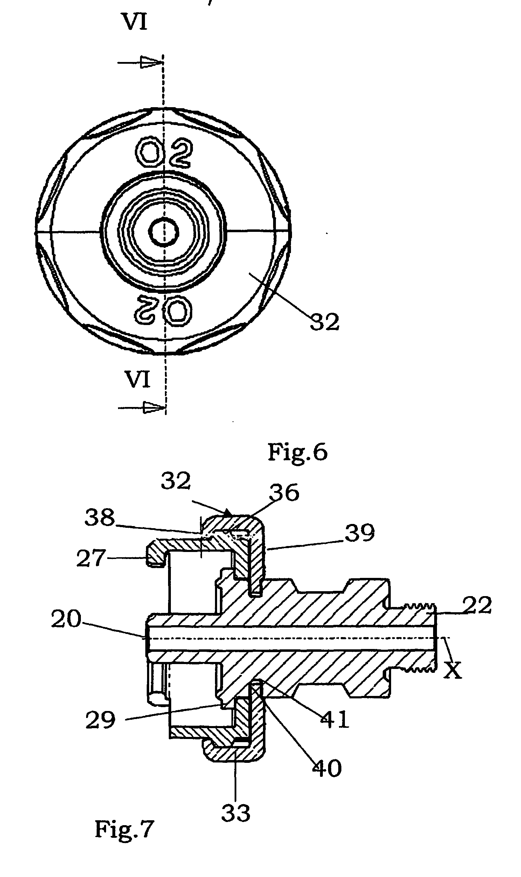

The shell 32 has a substantially C-shaped section (figure 7) and is formed by a mantle 33, a first end section 38 turned towards the first end 20 of the sleeve 18, and a second end section 39, having a greater extension with respect to the first end section 38, and turned towards the second end 22 of the sleeve 18. The mantle 33, the first end section 38 and the second end section 39 define an inner annular recess, in which a portion of the fastening element 24 is housed.

The second end section 39 also has an edge 40 having reduced thickness with respect to the whole shell 32, which is received in a circular groove 41 made on the outer surface of the sleeve 18. By means of the circular groove 41, the shell 32 is axially retained on the sleeve 18.

It is also shown that in order to favour handling, the shell 32 has a many-sided outer face, i.e. it is provided with a plurality of sides 43.

The shell 32 is restrained to the fastening element 24 by means of inner teeth, not visible in the figures, which are engaged with counter-teeth 36 of the bayonet clutch 24. The counter-teeth 36 of the bayonet clutch 24 are arranged along the entire outer circumference of the latter, forming a toothed rim, while groups of teeth are provided on the shell 32, which are arranged angularly offset by 180° along the inner surface of the mantle 33.

The entire counter-teeth rim 36 of the bayonet clutch 24 is housed and retained between the first end section 38 and the second end section 39 of the shell 32, in order to ensure a firm restraint between the two elements.

A circular flange 29 is also provided, made integrally with the sleeve 18 near the first end 20, flange 29 acting as an axial stop for the fastening element 24.

The fastening element 24 is therefore axially retained between the first end section 38 of the shell 32 and the circular flange 29 of the sleeve 18.

In the connection device 10, the shell 32 is formed by two opposite half-shells 44 and 46 having semi-circular form with an inner circumference and an outer circumference.

The half-shells 44 and 46 are provided at the respective ends 52, 54 with teeth 48, 49 adapted to be snap coupled into corresponding slots 50, 51 made on the opposite end of each half-shell 44 and 46. In particular, each half-shell 44, 46 has a first coupling tooth 48 arranged at the first end S2 on the inner circumference of the aforesaid semicircle, and a second coupling tooth 49 arranged on the other end 54 on the outer circumference of the semicircle. This arrangement of the teeth 48, 49 on the outer and inner circumferences of the two half-shells 44, 46 favours a stable and unequivocal reciprocal coupling of the two half-shells 44, 46.

In order to favour the coupling and the centring of the two half-shells 44, 46 in a correct position, each half-shell 44, 46 bears, at the first end 52, a projecting pin 56 destined to be inserted in a corresponding blind hole 58 made on the second end 54 of the other half-shell.

According to another aspect, each half-shell 44, 46 can have the same colour or a different colour from each other, in order to favour the recognition of the gas for which the device is destined, in accordance with pertaining laws. Moreover, each half-shell 44, 46 have writing indicating the symbol and/or name of the compressed gas or vacuum.

The assembly of the connection device 10 according to the present invention is very simple and is carried out in the following manner.

First, the fastening element 24 is inserted on the sleeve 18 until it abuts against the circular flange 29. Then, the two half-shells 44, 46 are reciprocally shut by means of respective teeth 48, such that the edge 40 is inserted in the corresponding circular groove 41 of the sleeve. The fastening element 24 is then shut between the two half-shells 44, 46.

By means of these two operations, the connection device 10 is then ready to be fixed to the apparatus for medical use 12 through screwing. Alternatively, the two half-shells can be fixed to the fastening element after the fixing of the sleeve to the apparatus for medical use 12.

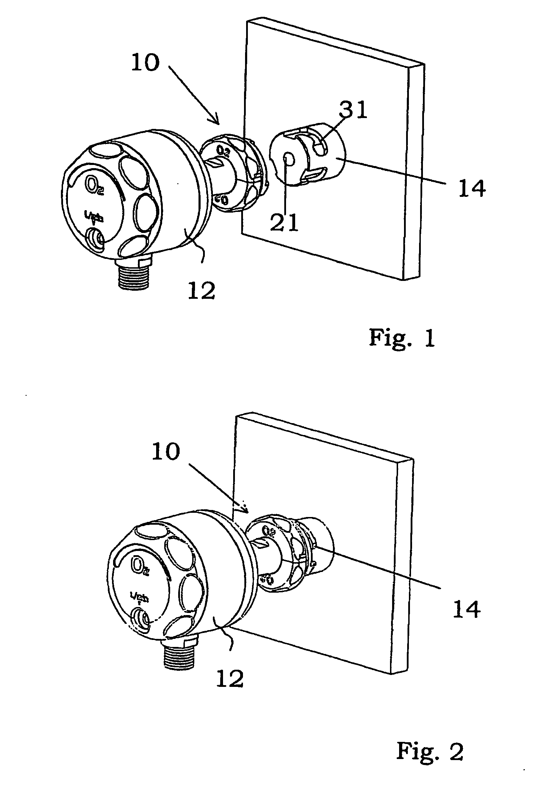

Subsequently, as illustrated in figure 2, the connection device 10 with the apparatus for medical use is fixed to the end unit. For such purpose, the first end 20 of the sleeve 18 is inserted in the hole 21 of the end unit, and the fastening element 24 is axially translated on the end unit 14 and subsequently angularly moved, such that the front teeth 27 slide in the grooves 31, until the attainment of the final position in the angular section of the grooves 31.

The main advantage of the present invention lies in the fact that, due to the idle condition of the single body formed by the fastening element and the handle element, the angular movement of the fastening element in order to achieve the fixing with the end unit is completely unconstrained by the sleeve and thus also by the apparatus for medical use, and thus a possible angular displacement of the apparatus for medical use is avoided. In substance, the apparatus for medical use is not constrained in the angular movement around the axis of the sleeve at the time of the fixing of the fastening element to the end unit.

In this manner, in the case of a plurality of apparatuses for medical use arranged alongside each other on a wall or on a movable trolley, one avoids risking that their mounting and disassembly is obstructed by adjacent apparatuses.

Moreover, if the apparatus is inadvertently angularly moved during the fixing to the end unit, a repositioning of the apparatus into correct positioning can be easily carried out by simply rotating the sleeve around its axis; said movement does not induce angular stress on the wall outlet, with consequent risk of disassembly from its own base.

Additional operations for the repositioning of the apparatus for medical use are thus unnecessary.

A further advantage lies in the fact that the angular movement of the fastening element is much facilitated for the user, it being unconstrained by the apparatus.

A further advantage lies in the two half-shell conformation of the handle element. The two half-shells can in fact be reciprocally assembled and shut on the handle element, even when the sleeve is already fixed to the apparatus for medical use.

## Claims

1. Connection device for connecting an apparatus for medical use to an end unit of a distribution plant of compressed gases or of a vacuum distribution plant, wherein the connection device is adapted to be fixed to the end unit (14) by means of a bayonet clutch having a fixed part and a movable part, of which the end unit constitutes the fixed part, and wherein the connection device is of the type comprising a sleeve (18) having tubular body extended along a specific axis (X) between a first end (20) adapted to be associated with the end unit (14) and a second end (22) adapted to be associated with the apparatus for medical use (12) for the passage of the compressed gas or vacuum, and also having a fastening element (24) constituting the movable part of the bayonet clutch, and a handle element (26) associated with the fastening element (24), **characterised in that** the fastening element (24) and the handle element (26) are rigidly bound to each other jointly forming a piece rotatably and idly mounted on the sleeve (18) and separately manoeuvrable with respect to the sleeve (18), the handle element (26) comprising an annular shell (32), which surrounds the fastening element (24), the shell (32) being formed by two opposite half-shells (44, 46) having semicircular form mounted around the fastening element (24).

2. Connection device according to claim 1, **characterised in that** the shell (32) has a substantially C-shaped section and is formed by a mantle (33), a first end section (38) turned towards the first end (20) of the sleeve (18), and a second end section (39) having greater extension with respect to the first end section (38), and turned towards the second end (22) of the sleeve (18), wherein the mantle (33), the first end section (38) and the second end section (39) define an inner annular recess in which a portion of the fastening element (24) is housed.

3. Connection device according to claim 2, **characterised in that** the fastening element (24) is axially retained between the first end (20) of the sleeve (18) and the second end section (39) of the handle element (26).

4. Connection device according to claim 3, **characterised in that** the second end section (39) has an edge (40) having a reduced thickness with respect to the whole shell (32), wherein the edge (40) is received in a circular groove (41) made on the outer surface of the sleeve (18).

5. Connection device according to claim 3, **characterised in that** it comprises a circular flange (29) made integrally with the sleeve (18) near the first end (20), which acts as an axial stop for the fastening element (24).

6. Connection device according to claim 1, **characterised in that** the shell (32) is constrained to the fastening element (24) by means of teeth which are engaged with counter-teeth (36) of the fastening element (24).

7. Connection device according to claim 6, **characterised in that** the counter-teeth (36) of the fastening element (24) are arranged on the entire outer circumference of the latter, forming a toothed rim.

8. Connection device according to claim 6, **characterised in that** groups of teeth of the shell (32) are arranged angularly offset by 90° along the inner surface of the mantle (33).

9. Connection device according to claim 7, **characterised in that** the entire rim of counter-teeth (36) of the fastening element (24) is received and retained between the first end section (38) and the second end section (39) of the shell (32), in order to ensure a firm restraint between the two elements.

10. Connection device according to claim 1, **characterised in that** the half-shells are provided at the respective ends (52, 54) with teeth (48, 49), adapted to be snap-coupled into corresponding slots (50, 51) made on the other end of each half-shell (44, 46).

11. Connection device according to claim 10, **characterised in that** each half-shell (44, 46) has a first coupling tooth (48) arranged at the first end (52) on the inner circumference of the semicircle, and a second coupling tooth (49) arranged on the other end (54) on the outer circumference of the semicircle.

12. Connection device according to claim 1, **characterised in that** each half-shell (44, 46) bears, at the first end (52), a projecting pin (56) intended to be inserted in a corresponding blind hole (58) made on the second end (54) of the other half-shell.

13. Connection device according to claim 12, **characterised in that** each half-shell can have colour identical to or different from the other half-shell.

14. Apparatus for medical use comprising a connection device according to any one of claims 1-13.

15. Supply device of a distribution plant of compressed gases or of a vacuum distribution plant comprising an end supply unit constituting the fixed part of a bayonet clutch and a connection device according to any one of claims 1-13.

## Patentansprüche

1. Anschlussvorrichtung zum Verbinden eines Geräts für medizinische Zwecke mit einem Endanschluss einer Verteilungsanlage von verdichteten Gasen oder einer Vakuum-Verteilungsanlage, wobei die Anschlussvorrichtung mittels einer Bajonettkupplung an einen Endelement (14) befestigbar ist, aufweisend einen ortsfesten und einen beweglichen Teil, bei dem das Endelement den ortsfesten Teil bildet und wobei die Anschlussvorrichtung eine Muffe (18) aufweist, die einen röhrenförmigen Körper hat, der sich entlang einer spezifischen Achse (X) zwischen einem ersten Ende (20), das mit dem Endelement (14) verbunden ist und einem zweiten Ende (22), das mit dem Gerät für medizinische Zwecke (12) für den Durchlass des verdichteten Gases verbunden ist, erstreckt, und weist zudem ein Verbindungselement (24) auf, das den beweglichen Teil der Bajonettkupplung bildet, und ein Griffelement (26), das mit dem Verbindungselement (24) verbunden ist, **dadurch gekennzeichnet, dass** das Verbindungselement (24) und das Griffelement (26) starr miteinander verbunden sind und ein Teil bilden, dass drehbar und träge auf der Muffe (18) befestigt ist und im Hinblick auf die Muffe (18) separat beweglich ist, das Griffelement (26) weist eine ringförmige Schale (32) auf, die das Verbindungselement (24) umgibt, die Schale (32) ist durch zwei gegenüberliegende Halbschalen (44, 46) gebildet, die eine halbrunde Form haben und um das Verbindungselement (24) angeordnet sind.

2. Anschlussvorrichtung nach Anspruch 1, **dadurch gekennzeichnet, dass** die Schale (32) einen im Wesentlichen c-förmigen Bereich hat und durch eine Hülle (33), einen ersten Endabschnitt (38), der zum ersten Ende (20) der Muffe (18) zeigt, und einen zweiten Endabschnitt (39), der im Vergleich zum ersten Endabschnitt (38) eine größere Ausdehnung aufweist und zum zweiten Ende (22) der Muffe (18) zeigt, gebildet ist, wobei die Hülle (33), der erste Endabschnitt (38) und der zweite Endabschnitt (39) eine innere ringförmige Vertiefung definieren, in der ein Teil des Verbindungselements (24) untergebracht ist.

3. Anschlussvorrichtung nach Anspruch 2, **dadurch gekennzeichnet, dass** das Verbindungselement (24) axial zwischen dem ersten Ende (20) der Muffe (18) und dem zweiten Endabschnitt (39) des Griffelements (39) gehalten wird.

4. Anschlussvorrichtung nach Anspruch 3, **dadurch gekennzeichnet, dass** der zweite Endabschnitt (39) einen Vorsprung (40) umfasst, der im Vergleich zu der gesamten Schale (32) eine reduzierte Dicke hat, wobei der Vorsprung (40) in einer umlaufenden Nut (41) aufgenommen ist, die auf der äußeren Fläche der Muffe (18) angeordnet ist.

5. Anschlussvorrichtung nach Anspruch 3, **dadurch gekennzeichnet, dass** sie einen Rundflansch (29) aufweist, der in der Nähe des ersten Endes (20) einen Teil der Muffe (18) ausmacht und der als ein axialer Anschlag für das Verbindungselement (24) dient.

6. Anschlussvorrichtung nach Anspruch 1, **dadurch gekennzeichnet, dass** die Schale (32) mittels Zähne, die im Eingriff mit den Widerzähnen (36) des Verbindungselements (24) stehen, mit dem Verbindungselement (24) verbunden ist.

7. Anschlussvorrichtung nach Anspruch 6, **dadurch gekennzeichnet, dass** die Widerzähne (36) des Verbindungselements (24) auf dem gesamten Außenumfang des Verbindungselements (24) angeordnet sind, um einen Zahnkranz zu bilden.

8. Anschlussvorrichtung nach Anspruch 6, **dadurch gekennzeichnet, dass** Gruppen der Zähne der Schale (32) eckig um 90° versetzt entlang der inneren Fläche der Hülle (33) angeordnet sind.

9. Anschlussvorrichtung nach Anspruch 7, **dadurch gekennzeichnet, dass** der komplette Kranz der Widerzähne (36) des Verbindungselements (24) zwischen dem ersten Endabschnitt (38) und dem zweiten Endabschnitt (39) der Schale (32) festgehalten wird, um eine feste Verspannung zwischen den beiden Elementen sicherzustellen.

10. Anschlussvorrichtung nach Anspruch 1, **dadurch gekennzeichnet, dass** an den Halbschalen an den jeweiligen Enden (52, 54) Zähne (48, 49) vorgesehen sind, um in entsprechende Aussparungen (50, 51), die am anderen Ende jeder Halbschale (44, 46) angeordnet sind, einzurasten.

11. Anschlussvorrichtung nach Anspruch 10, **dadurch gekennzeichnet, dass** jede Halbschale (44, 46) einen ersten Verbindungszahn (48) aufweist, der an dem ersten Ende (52) am inneren Umfang des Halbkreises angeordnet ist und einen zweiten Verbindungszahn (49) aufweist, der an dem anderen Ende (54) am äußeren Umfang des Halbkreises angeordnet ist

12. Anschlussvorrichtung nach Anspruch 1, **dadurch gekennzeichnet, dass** jede Halbschale (44, 46) an dem ersten Ende (52) einen Stift (56) aufweist, um in ein entsprechendes Sackloch (58) des zweiten Endes (54) der anderen Halbschale eingefügt zu werden.

13. Anschlussvorrichtung nach Anspruch 12, **dadurch gekennzeichnet, dass** jede Halbschale dieselbe oder eine andere Farbe als die andere Halbschale haben kann.

14. Vorrichtung für einen medizinischen Einsatz mit einer Anschlussvorrichtung nach einem der Ansprüche 1-13.

15. Zuführeinrichtung einer Verteilungsanlage von verdichteten Gasen und einer Vakuum-Verteilungsanlage aufweisend eine End-Versorgungseinrichtung, die den ortsfesten Teil einer Bajonettkupplung und einer Anschlussvorrichtung nach einem der Ansprüche 1 bis 13 bildet.

## Revendications

1. Dispositif de raccord pour raccorder un appareil destiné à une utilisation médicale à une unité terminale d'une installation de distribution de gaz comprimés ou d'une installation de distribution de dépression, dans lequel le dispositif de raccord est adapté pour être fixé à l'unité terminale (14) au moyen d'une fermeture à baïonnette ayant une partie fixe et une partie mobile, dont l'unité terminale constitue la partie fixe, et où le dispositif de raccord est du type comprenant un manchon (18) ayant un corps tubulaire allongé le long d'un axe spécifique (X) entre une première extrémité (20) adaptée pour être associée à l'unité terminale (14) et une seconde extrémité (22) adaptée pour être associée avec l'appareil destiné à une utilisation médicale (12) pour le passage du gaz comprimé ou de la dépression, et ayant également un élément de fixation (24) constituant la partie mobile de la fermeture à baïonnette, et un élément de préhension (26) associé à l'élément de fixation (24), **caractérisé en ce que** l'élément de fixation (24) et l'élément de préhension (26) sont liés solidement l'un à l'autre formant ensemble une pièce montée de manière rotative et libre sur le manchon (18) et pouvant être manoeuvrée séparément par rapport au manchon (18), l'élément de préhension (26) comprenant une coque annulaire (32), qui entoure l'élément de fixation (24), la coque (32) étant formée par deux demi-coques opposées (44, 46) ayant une forme hémicirculaire montée autour de l'élément de fixation (24).

2. Dispositif de raccord selon la revendication 1, **caractérisé en ce que** la coque (32) présente une coupe sensiblement en forme de C et est formée par un manteau (33), une première section d'extrémité (38) tournée vers la première extrémité (20) du manchon (18), et une seconde section d'extrémité (39) présentant une extension plus longue que la première section d'extrémité (38), et tournée vers la seconde extrémité (22) du manchon (18), où le manteau (33), la première section d'extrémité (38) et la seconde section d'extrémité (39) définissent un évidement annulaire intérieur dans lequel une partie de l'élément de fixation (24) est logée.

3. Dispositif de raccord selon la revendication 2, **caractérisé en ce que** l'élément de fixation (24) est retenu de manière axiale entre la première extrémité (20) du manchon (18) et la seconde section d'extrémité (39) de l'élément de préhension (26).

4. Dispositif de raccord selon la revendication 3, **caractérisé en ce que** la seconde section d'extrémité (39) possède un bord (40) ayant une épaisseur réduite par rapport à la coque complète (32), où le bord (40) est reçu dans une rainure circulaire (41) réalisée sur la surface extérieure du manchon (18).

5. Dispositif de raccord selon la revendication 3, **caractérisé en ce qu'**il comprend une bride circulaire (29) faite d'un seul tenant avec le manchon (18) à proximité de la première extrémité (20), qui agit comme une butée axiale pour l'élément de fixation (24).

6. Dispositif de raccord selon la revendication 1, **caractérisé en ce que** la coque (32) est maintenue de manière contraignante sur l'élément de fixation (24) au moyen de dents qui sont mises en prise avec des contre-dents (36) de l'élément de fixation (24).

7. Dispositif de raccord selon la revendication 6, **caractérisé en ce que** les contre-dents (36) de l'élément de fixation (24) sont agencées sur l'entière circonférence extérieure de ce dernier, formant une couronne dentée.

8. Dispositif de raccord selon la revendication 6, **caractérisé en ce que** des groupes de dents de la coque (32) sont agencés avec un décalage angulaire de 90° le long de la surface intérieure du manteau (33).

9. Dispositif de raccord selon la revendication 7, **caractérisé en ce que** la totalité de la couronne de contre-dents (36) de l'élément de fixation (24) est reçue et retenue entre la première section d'extrémité (38) et la seconde section d'extrémité (39) de la coque (32), afin d'assurer une retenue solide entre les deux éléments.

10. Dispositif de raccord selon la revendication 1, **caractérisé en ce que** les demi-coques sont munies à leurs extrémités respectives (52, 54) de dents (48, 49), adaptées pour être couplées par encliquetage dans des fentes correspondantes (50, 51) réalisées sur l'autre extrémité de chaque demi-coque (44, 46).

11. Dispositif de raccord selon la revendication 10, **caractérisé en ce que** chaque demi-coque (44, 46) possède une première dent de couplage (48) agencée à la première extrémité (52) sur la circonférence intérieure du demi-cercle, et une seconde dent de couplage (49) agencée sur l'autre extrémité (54) sur la circonférence extérieure du demi-cercle.

12. Dispositif de raccord selon la revendication 1, **caractérisé en ce que** chaque demi-coque (44, 46) porte, à la première extrémité (52), une broche faisant saillie (56) destinée à être insérée dans un trou borgne correspondant (58) réalisé sur la seconde extrémité (54) de l'autre demi-coque.

13. Dispositif de raccord selon la revendication 12, **caractérisé en ce que** chaque demi-coque peut avoir une couleur identique ou différente de l'autre demi-coque.

14. Appareil destiné à une utilisation médicale comprenant un dispositif de raccord selon l'une quelconque des revendications 1 à 13.

15. Dispositif d'alimentation d'une installation de distribution de gaz comprimés ou d'une installation de distribution de dépression comprenant une unité terminale d'alimentation constituant la partie fixe d'une fermeture à baïonnette et un dispositif de raccord selon l'une quelconque des revendications 1 à 13.
